# EUROPEAN PATENT SPECIFICATION

(11) **EP 1 137 433 B1**
(45) Date of publication and mention of the grant of the patent: **07.05.2003**
(21) Application number: 99957333.0
(22) Date of filing: 02.12.1999
(51) Int. Cl.: A61K 38/52, A61K 39/00, C12N 9/90, A61P 35/00

(54) **PHARMACEUTICAL COMPOSITIONS CONTAINING PROTEIN-DISULFIDE ISOMERASES**
PROTEIN-DISULFID-ISOMERASEN ENTHALTENDE PHARMAZEUTISCHE ZUSAMMENSETZUNGEN
COMPOSITIONS PHARMACEUTIQUES CONTENANT DES PROTEINES

(30) Priority: 04.12.1998 IT MI982634
(43) Date of publication of application: 04.10.2001
(73) Proprietor: Pharmaproducts UK Limited, Liverpool, Merseyside L2 9TL (GB)
(72) Inventor: BARTORELLI, Alberto, 3963 Crans Sur Sierre (CH)
(74) Representative: Minoja, Fabrizio, Dr.
(86) International application number: EP9909398
(87) International publication number: WO00033861

(56) References cited:
- WO-A-98/45416
- WO-A-99/29834
- WO-A-99/49038
- US-A- 5 798 249
- LUNDSTRÖM-LJUNG J ET AL: "Two resident ER-proteins, CaBP1 and CaBP2, with thioredoxin domains, are substrates for thioredoxin reductase: comparison with protein disulfide isomerase" FEBS LETTERS, vol. 357, 1995, pages 305-308, XP002138843
- BASU SREYASHI ET AL: "Protein disulfide isomerase, a peptide binding chaperone of the endoplasmic reticulum, elicits tumor specific and peptide specific immunity." FASEB JOURNAL, vol. 12, no. 4, 17 March 1998 (1998-03-17), page A588 XP002138844 ISSN: 0892-6638
- URADE R ET AL: "Protein degradation by ERp72 from rat and mouse liver endoplamic reticulum" THE JOURNAL OF BIOLOGICAL CHEMISTRY, vol. 268, no. 29, 15 October 1993 (1993-10-15), pages 22004-22009, XP002138845 cited in the application

## Description

The present invention relates to particular protein-disulfide isomerases in connection with the therapy and the prophylaxis of neoplastic pathologies.

Protein-disulfide isomerases are a family of enzymes which, in addition to not yet completely elucidated functions, have endoproteinase and chaperonin activities.

The endoproteinase activity can be modulated by calcium ions and is inhibited by Cys-protease inhibitors and is therefore similar to that of other known Cys-proteases, such as calpain, with which no significant sequence homologies exist, apart from the thioredoxin CGHC motif common to many Cys-proteases.

The chaperonin activity has been evidenced in the refolding of the Fab fragment of some monoclonal antibodies and cannot be modulated by calcium ions.

Protein-disulfide isomerases catalyse the formation of disulfide bonds among protein chains and are therefore involved in the "folding" processes of proteins thus playing an important role in the maintenance of fundamental cellular processes.

Examples of protein-disulfide isomerases are the ERp72 endoplasmic reticule proteins (from humans, rat and mouse) described in J. Biol. Chem. 268(29), 22004-22009; 1993; ibidem 269(4), 2501-2507, 1994; ibidem 266, 5353, 1991 and the P5 protein from hamster liver described in Biochem. J. 281, 645-650, 1992.

Rupp et al. (J. Biol. Chem. 269(4), 2501-2507, 1994) have defined some of these proteins with the abbreviations CaBP1 and CaBP2, from "Calcium binding protein".

Other members of the PDI family comprise the proteins known as calsequestrin, BpI, Erp60.

WO 9845416 (October 15, 1998) discloses the use of thioredoxine like protein (TIOP) for the treatment of disorders associated with the expression of said protein, including cancer.

It has now been found that protein-disulfide isomerases (PDI) and related proteins selected from ERp72, ERp60, P5 and calsequestrin, when injected subcutaneously in Balb/c mice and in rabbits, induce an antibody response of IgM type characterized by a cytotoxicity which can be evidenced in vitro on human tumor cell lines, such as the Jurkat and Kato III lines. The invention therefore relates to said protein-disulfide isomerases as prophylactic and therapeutical agents, in particular as antitumor agents.

The same immunological and cytotoxic properties have been observed in a protein isolated from goat liver with procedures similar to those described for the proteins cited above. Said protein has molecular weight of about 72 Kda in SDS-PAGE and a high sequence homology (≥90%) to the ERp72 proteins.

Said protein is obtainable by extraction of mammals liver with PBS followed by purification by chromatography on hydrophobic exchange gel columns.

The process for the extraction and purification of the protein of the invention, in the following referred to as p72, is illustrated in the annexed Figure.

Cytotoxicity is quantifiable in vitro on Jurkat and Kato III cells using conventional methods, based on, for example, the use of commercial kits such as the CDCµK kit (Pharmaproduct). In particular, cytotoxicity was observed in rabbit serum already after a first treatment with p72 (1 mg/animal in saline solution) on Jurkat and Kato III cells. Cytotoxicity remained steady or increased one month after a second treatment effected two weeks after the first, with values sometimes reaching 85%-89%.

PDIs or p72 are useful as therapeutical or prophylactic agents against tumors of various origin, in particular carcinomas and adenocarcinomas.

PDIs or p72 will be administered at dosages ranging from 1 to 10 mg/patient, using the conventional administration routes for proteins and polypeptides, for example the subcutaneous or intramuscular routes. The treatment can be repeated and a treatment comprising one-two week spaced administrations is preferable.

Furthermore, it has surprisingly been found that high cytotoxicity can also be induced by administering PDIs or p72 at very low dosages, of the order of 1 . 10⁻⁴ - 1 . 10⁻¹⁰ g, sublingually, in the form of granules or drops of 1% ethanol water-alcoholic solutions or suspensions, with concentrations of active ingredient ranging from 10⁻⁶ to 10⁻¹⁰ M.

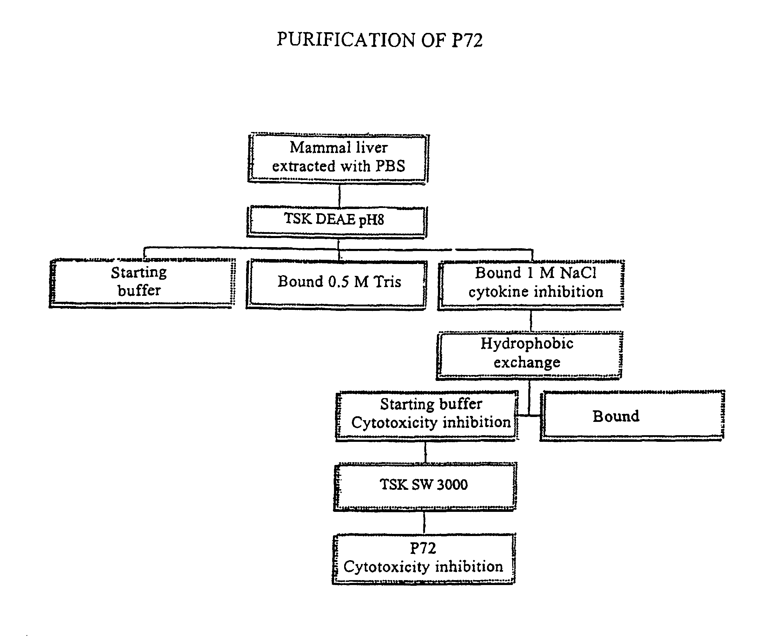

## Claims

1. Protein-disulfide isomerases selected from ERp72, ERp60, P5, calsequestrin as therapeutical agents.

2. Pharmaceutical compositions containing the proteins of claim 1 as active ingredient.

3. Antitumoral vaccines containing the proteins of claim 1 as active ingredient.

4. Compositions as claimed in claim 2 or 3 in a form suitable to the parenteral or oral-sublingual administrations.

5. Compositions as claimed in claim 4, as sterile injectable solutions, drops or granules.

6. Antitumoral vaccines containing the proteins of claims 1-4 as active ingredient.

7. Compositions as claimed in claim 5 or 6 in a form suitable to the parenteral or oral-sublingual administrations.

8. Compositions as claimed in claim 7, as sterile injectable solutions, drops or granules.

## Patentansprüche

1. Protelndisulfid-lsomerasen, ausgewählt aus ERp72, ERp60, P5, Calsequestrin als therapeutische Mittel.

2. Pharmazeutische Zusammensetzungen, enthaltend die Proteine nach Anspruch 1 als aktiven Bestandteil.

3. Antitumor-Impfstoffe, enthaltend die Proteine nach Anspruch 1 als aktiven Bestandteil.

4. Zusammensetzungen wie in Anspruch 2 oder 3 beansprucht in einer für die parenterale oder oral-sublinguale Gabe geeigneten Form.

5. Zusammensetzungen wie in Anspruch 4 beansprucht als sterile injizierbare Lösungen, Tropfen oder Granulate.

6. Antitumor-Impfstoffe, enthaltend die Proteine nach den Ansprüchen 1 bis 4 als aktiven Bestandteil.

7. Zusammensetzungen wie in Anspruch 5 oder 6 beansprucht in einer für die parenterale oder oral-sublinguale Gabe geeigneten Form.

8. Zusammensetzungen wie in Anspruch 7 beansprucht als sterile injizierbare Lösungen, Tropfen oder Granulate.

## Revendications

1. Protéine-disulfure isomérases choisies parmi ERp72, ERp60, P5, calséquestrine, en tant qu'agents thérapeutiques.

2. Compositions pharmaceutiques contenant les protéines de la revendication 1 en tant qu'ingrédient actif.

3. Vaccins antitumoraux contenant les protéines de la revendication 1 en tant qu'ingrédient actif.

4. Compositions telles que revendiquées dans la revendication 2 ou 3 sous une forme appropriée pour des administrations par voie parentérale ou orale-sublinguale.

5. Compositions telles que revendiquées dans la revendication 4, en tant que solutions injectables, gouttes et granulés stériles.

6. Vaccins antitumoraux contenant les protéines des revendications 1 à 4 en tant qu'ingrédient actif.

7. Compositions telles que revendiquées dans la revendication 5 ou 6 sous une forme appropriée pour des administrations par voie parentérale ou orale-sublinguale.

8. Compositions telles que revendiquées dans la revendication 7, en tant que solutions injectables, gouttes et granulés stériles.
